(19) **Europäisches Patentamt**

**European Patent Office**

**Office européen des brevets**

(11) **EP 0 648 105 B1**

(12) **EUROPÄISCHE PATENTSCHRIFT**

(45) Veröffentlichungstag und Bekanntmachung des Hinweises auf die Patenterteilung:
**03.06.1998 Patentblatt 1998/23**

(21) Anmeldenummer: **94912530.6**

(22) Anmeldetag: **24.03.1994**

(51) Int. Cl.$^6$: **A61K 7/06**

(86) Internationale Anmeldenummer:
**PCT/EP94/00936**

(87) Internationale Veröffentlichungsnummer:
**WO 94/26235 (24.11.1994 Gazette 1994/26)**

(54) **HAARBEHANDLUNGSMITTEL**

HAIR CARE PRODUCT

PRODUIT DE SOINS DES CHEVEUX

(84) Benannte Vertragsstaaten:
**DE ES FR GB IT**

(30) Priorität: **08.05.1993 DE 4315405**

(43) Veröffentlichungstag der Anmeldung:
**19.04.1995 Patentblatt 1995/16**

(73) Patentinhaber:
**Wella Aktiengesellschaft**
**64274 Darmstadt (DE)**

(72) Erfinder:
• **CLAUSEN, Thomas, Dr.**
**D-64665 Alsbach (DE)**
• **LANG, Günther, Dr.**
**D-64354 Reinheim (DE)**
• **KEIL, Wolfgang, Dr.**
**D-63165 Mühlheim (DE)**
• **FRANZKE, Michael, Dr.**
**D-64380 Rossdorf (DE)**
• **SCHMENGER, Jürgen**
**D-64331 Weiterstadt (DE)**
• **SCHONERT, Dieter**
**D-64354 Reinheim (DE)**

(56) Entgegenhaltungen:
| | |
|---|---|
| EP-A- 0 034 190 | EP-A- 0 274 086 |
| EP-A- 0 370 764 | GB-A- 2 136 689 |
| GB-A- 2 184 449 | GB-A- 2 206 045 |
| GB-A- 2 222 949 | |

Bemerkungen:
Die Akte enthält technische Angaben, die nach dem Eingang der Anmeldung eingereicht wurden und die nicht in dieser Patentschrift enthalten sind.

**Beschreibung**

Gegenstand der Erfindung ist ein Haarbehandlungsmittel auf wäßriger Basis, welches einen Verdicker und ein festigendes wasserlösliches Polymer, das durch Änderung des pH-Wertes ganz oder teilweise ausfällbar ist, enthält und einen pH-Wert aufweist, bei dem das festigende Polymer ganz oder teilweise ausgefällt vorliegt.

Übliche Haarbehandlungsmittel mit einem Gehalt an natürlichen, synthetischen oder modifizierten natürlichen filmbildenden Polymeren liegen meist in Form alkoholischer, wäßrig-alkoholischer oder in einer ein anderes organisches Lösungsmittel enthaltenden Zubereitung vor.

Organische Lösungsmittel in Haarpflegeprodukten wirken bei täglichem Gebrauch belastend auf Mensch und Umwelt und haben zudem einen unangenehmen Geruch. Ersetzt man in solchen Mitteln das organische Lösungsmittel durch Wasser, so ist dies meist mit einer Verschlechterung der Gebrauchseigenschaften, wie zum Beispiel einem langsameren Trocknen der Haare, verbunden. Die Haare fühlen sich in diesen Fällen meist klebrig an.

Haarbehandlungsmittel mit stark festigender Wirkung enthalten hohe Anteile eines oder mehrerer filmbildender festigender Polymere, wodurch bei der Anwendung das Haar, insbesondere feines Haar, stark belastet wird. Darüber hinaus kann das Haar leicht verkleben.

Die gebräuchlichen Formen solcher Mittel sind heute Sprays, Lotionen, Schäume und Gele. Insbesondere bei Gelen treten die vorstehend genannten Probleme häufig auf.

Darüber hinaus ist die Festigungsleistung von Haarbehandlungsmitteln in Gelform begrenzt.

Aus der EP-OS 0 445 714 sind haarfestigende Mittel bekannt, die einen Gehalt an Natriumalginat, einem festigenden Polymer und Wasser als Lösungsmittel aufweisen. Die Festigungsleistung solcher Mittel ist jedoch begrenzt. Ferner kann es zu einer Belastung des Haares kommen.

In der EP-OS 0 412 705 ist ein haarkosmetisches Mittel offenbart, welches ein nichtionisches, wasserlösliches, hydrophob modifiziertes verdickendes Polymer, ein zweites wasserlösliches verdickendes Polymer und ein wasserlösliches oder wasserunlösliches haarfestigendes Polymer enthält. Als bevorzugte festigende Polymere werden spezielle Silikon-Copolymere genannt, die in flüssigen Silikonderivaten gelöst, in das Mittel eingearbeitet werden. Aufgrund des Einsatzes von Silizium-organischen Lösungsmitteln sind derartige Mittel bezüglich der Umweltverträglichkeit nicht zufriedenstellend.

Aufgabe war es daher, ein Haarbehandlungsmittel zur Verfügung zu stellen, welches die vorstehend genannten Nachteile nicht aufweist.

Überraschenderweise wurde nunmehr gefunden, daß die gestellte Aufgabe durch ein Haarbehandlungsmittel mit einem Gehalt an

| | | |
|---|---|---|
| (A) 0,1 bis 30 | Gewichtsprozent mindestens eines organischen und/oder anorganischen Verdickers | |
| (B) 0,1 bis 25 | Gewichtsprozent mindestens eines festigenden wasserlöslichen Polymers, das durch Änderung des pH-Wertes ganz oder teilweise ausfällbar ist, und | |
| (C) 45 bis 99,8 | Gewichtsprozent Wasser, welches, | |
| (D) | frei von organischen Lösungsmitteln ist und | |
| (E) | einen pH-Wert aufweist, bei dem die Komponente (B) ganz oder teilweise ausgefällt vorliegt, in hervorragender Weise gelöst wird. | |

Das Haarbehandlungsmittel enthält die Komponente (A) vorzugsweise in einer Menge von 0,15 bis 15 Gewichtsprozent. Die Verdicker der Komponente (A) können ausgewählt sein aus der Klasse der natürlichen, modifizierten natürlichen und/oder synthetischen Verdicker. Beispiele für bevorzugte synthetische Verdicker sind unter anderem Polymere und Copolymere der Acrylsäure, Polymere und Copolymere der Methacrylsäure, Polymere und Copolymere der Crotonsäure oder Polymere und Copolymere der Salze oder Ester der Acrylsäure, Methacrylsäure oder Crotonsäure, wie zum Beispiel Polyacrylsäure (CTFA-Carbomer), wie sie unter den Handelsnamen Carbopol® der Firma B.F. Goodrich (USA) oder Acrisint® der Firma Sigma (Italien) erhältlich sind, Copolymere aus Acrylamid und Natriumacrylat (CTFA-Acrylamide/Sodium Acrylate Copolymer), beispielsweise Hostacerin PN 73® der Firma Hoechst (Deutschland), Copolymere aus einem mit 20 Mol Ethylenoxid oxethylierten Stearylalkohol-Methacrylsäureester und Acrylsäure (CTFA-Acrylates/Steareth-20 Methacrylate Copolymer) zum Beispiel Acrysol ICS-1® der Firma Rohm und Haas (USA) sowie mit Dienen vernetzte Copolymere aus ungesättigten Säureanhydriden und Alkylvinylethern vom Typ PVM/MA Copolymer, zum Beispiel das mit Decadien vernetzte Methylvinylether-Maleinsäurehydrid-Copolymer Stabileze® 06 der Firma ISP (USA).

Ferner können als synthetische Verdicker Polyethylenglykole, Polypropylenglykole oder Polyethylenglykol-Polypropylenglykol-Copolymere verwendet werden.

Die Bezeichnungen der beispielhaft aufgeführten Polymere wurden dem CTFA Cosmetic Ingredient Dictionary, 4. Auflage, 1991, entnommen.

Bevorzugte natürliche oder modifiziert natürliche Verdicker sind beispielsweise Polysaccharide, deren Derivate oder deren Hydrolysate. Beispiele für derartige Verbindungen sind Cellulose und Cellulosederivate, Stärke und Stärkederivate, Chitin und Chitinderivate, Chitosan und Chitosanderivate, Alginsäure und Alginate, Carrageenan, Maltodextrin, Dextrin, Guargummi, Xanthangummi, Gellangummi sowie Hyaluronsäure und Hyaluronsäuresalze. Ferner können Verdicker wie Gelatine, Hectorite oder Bentonite verwendet werden.

Das Haarbehandlungsmittel enthält die Polymeren der Komponente (B) vorzugsweise in einer Menge von 0,2 bis 10 Gewichtsprozent. Als Verbindungen der Komponente (B) können beispielsweise filmbildende natürliche, modifizierte natürliche oder synthetische Polymere, die unter anderem aus Monomeren mit sauren, basischen oder sauren und basischen funktionellen Gruppen aufgebaut sind, verwendet werden.

Solche funktionellen Gruppen können unter anderem Carboxyl-, Hydroxyl-, Sulfo- oder Phosphatgruppen, deren Salze, freie oder substituierte Aminogruppen oder deren Salze sein. Das erfindungsgemäße Mittel enthält bevorzugt festigende Polymere, die in Form ihrer Salze wasserlöslich, als freie Base und/oder Säure dagegen wasserunlöslich sind. Sind sowohl saure als auch basische Gruppen im Polymer (amphotere Polymere) enthalten, ist dieses sowohl im sauren als auch im alkalischen Bereich löslich, so daß das Polymer nur am isoelektrischen Punkt ausfällbar ist. Der isoelektrische Punkt für solche Polymere liegt bevorzugt in einem pH-Bereich von 3 bis 8,5. Die in dem erfindungsgemäßen Mittel verwendeten synthetischen Copolymere, Pfropfcopolymere oder Terpolymere enthalten vorzugsweise als einen Monomerbaustein Verbindungen aus der Gruppe der ungesättigten Mono- oder Dicarbonsäuren oder der Anhydride von Dicarbonsäuren, wie zum Beispiel Methacrylsäure, Maleinsäure, Maleinsäureanhydrid oder Crotonsäure. Als weitere Monomerbausteine enthält das Polymer ungesättigte organische Verbindungen, vorzugsweise ungesättigte Carbonsäuren und/oder ihre Derivate, Vinylester und/oder ihre Verseifungsprodukte, Vinylamine, Vinylamide, Vinylether, Vinyllactone, Vinyllactame oder Vinylammoniumverbindungen. Beispiele für geeignete Co- oder Terpolymere sind das Octylacrylamid/Acrylsäure/Butylaminoethylmethacrylsäure-Terpolymer (CTFA-Octylacrylamide/Acrylates/Butylaminoethyl Methacrylate-Copolymer), zum Beispiel Amphomer®, National Starch (USA); Acrylsäure/Acrylamid-Copolymer (CTFA-Acrylates/Acrylamide Copolymer), zum Beispiel Ultrahold 8®, BASF (Deutschland); Vinylacetat/Crotonsäure-Copolymer (CTFA-VA/Crotonates Copolymer), zum Beispiel Luviset CA 66®, BASF, oder Resyn 28-1310®, National Starch (USA); Acrylsäure/Polyvinylpyrrolidon-Copolymer (CTFA-Acrylates/PVP Copolymer), zum Beispiel Luviflex VBM 35®, BASF; Methylvinylether/Maleinsäureanhydrid-Copolymer (CTFA-PVM/MA Copolymer), zum Beispiel vom Typ Gantrez®, GAF (USA), oder Vinylacetat/Crotonsäure/Vinylneodecanat-Copolymer (CTFA-VA/Crotonates/Vinyl Neodecanoate Copolymer), zum Beispiel Resyn 28-2930, National Starch (USA).

Die Bezeichnungen der beispielhaft aufgeführten Polymere wurden dem CTFA Cosmetic Ingredient Dictionary, 4. Auflage, 1991, entnommen.

Das Mittel enthält vorzugsweise 55 bis 96 Gewichtsprozent Wasser (Komponente (C)).

Der pH-Wert des Mittels liegt in einem Bereich von 1,5 bis 11,5 wobei ein pH-Wert von 2,5 bis 9,5 bevorzugt ist. Zur Einstellung des pH-Wertes können physiologisch verträgliche Säuren oder Basen, wie zum Beispiel Milchsäure, Zitronensäure, Glyoxylsäure, Ameisensäure, Essigsäure, Phosphorsäure, Aminomethylpropanol, Triisopropylamin, Natronlauge, Kalilauge, Ammoniak oder Monoethanolamin verwendet werden.

Weiterhin kann das erfindungsgemäße Mittel für Haarbehandlungsmittel übliche kosmetische Zusatzstoffe enthalten, wie zum Beispiel anionische, kationische, amphotere oder nichtionische Netzmittel und Emulgatoren wie oxethylierte Fettalkohole, Fettsäurealkanolamide, Alkyltrimethylammoniumsalze, Alkylbetaine, Alkylaminobetaine, Alkylsulfobetaine und Fettsäurealkylamidobetaine in einer Menge von 0,01 bis 20 Gewichtsprozent; Schaumsynergisten; Schaumstabilisatoren Sequestriermittel; Emulgatoren; Naturstoffe; Pigmente; Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Puffersubstanzen, wie beispielsweise Natriumcitrat oder Natriumphosphat, in einer Menge von 0,1 bis 1,0 Gewichtsprozent; sowie Farbstoffe, wie zum Beispiel Fluorescein-Natriumsalz, Gelb ZN3 (C.I. 47 055), in einer Menge von 0,1 bis 1,0 Gewichtsprozent; weiterhin haarpflegende Zusätze, wie zum Beispiel Fettsäureester, Fettalkohole, Fettsäureglyceride, ethoxylierte oder propoxylierte gesättigte Fettalkohole; Pflegestoffe, wie zum Beispiel Lanolinderivate, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; Antischuppenwirkstoffe; kosmetische Öle und Wachse sowie Konservierungsstoffe.

Die für Haarbehandlungsmittel typischen kosmetischen Zusatzstoffe können in einer Menge von 0,1 bis 20 Gewichtsprozent enthalten sein.

Das erfindungsgemäße Haarbehandlungsmittel kann in Form eines Haarpflegemittels, eines Shampoos oder eines Haarfestigungsmittels vorliegen. Es kann gegebenenfalls durch einen Gehalt an direkt auf das Haar aufziehenden Farbstoffen das Haar gleichzeitig färben oder tönen.

Derartige Präparate sind unter anderem als Farbfestiger oder Tönungsfestiger im Handel bekannt. Sie enthalten zusätzlich übliche direkt auf das Haar aufziehende Farbstoffe, beispielsweise aromatische Nitrofarbstoffe, zum Beispiel 1,4-Diamino-2-nitro-benzol, Pikraminsäure, 1-Hydroxy-2-amino-4-nitrobenzol und 1,4-bis(2-Hydroxyethyl)amino-2-nitro-5-chlorbenzol, Azofarbstoffe, zum Beispiel Acid Brown 4 (C.I. 14 805), Anthrachinonfarbstoffe, zum Beispiel Disperse Violet 4 (C.I. 61 105) und Triphenylmethanfarbstoffe, zum Beispiel Basic Violet 1 (C.I. 42 535), Basic Violet 14 (C.I. 42 510) oder Basic Blue 7 (C.I. 42 595:1), wobei die Farbstoffe dieser Klassen, je nach Art ihrer Substituenten, sauren, nichtionogenen oder basischen Charakter haben können. Ihre Gesamtkonzentration in dem erfindungsgemäßen Mittel beträgt üblicherweise etwa 0,01 bis 2,0 Gewichtsprozent.

Das Haarbehandlungsmittel kann in Form einer Lotion, einer Sprühlotion, eines Gels oder einer Creme vorliegen.

Das erfindungsgemäße Mittel kann weiterhin in einen Druckbehälter zusammen mit einem Treibmittel abgefüllt werden und aus diesem in Form eines Aerosolsprays oder Aerosolschaumes entnommen werden. Es kann auch in Form eines, mit Hilfe einer geeigneten mechanisch betriebenen Sprühvorrichtung versprühbaren Non-Aerosol-Haarsprays oder Non-Aerosol-Schaumes vorliegen.

Unter mechanischen Sprühvorrichtungen sind solche Vorrichtungen zu verstehen, welche das Versprühen einer Flüssigkeit ohne Verwendung eines verflüssigten Treibmittels ermöglichen. Als geeignete mechanische Sprühvorrichtungen kann beispielsweise eine Sprühpumpe oder ein mit einem Sprühventil versehener elastischer Behälter, in den das vorstehend beschriebene Mittel unter Druck abgefüllt wird, wobei sich der elastische Behälter ausdehnt und aus dem sich das Mittel infolge der Kontraktion des elastischen Behälters bei Öffnen des Sprühventils kontinuierlich entnehmen läßt, verwendet werden.

Wenn das erfindungsgemäße Mittel in Form eines Aerosol-Haarsprays oder Aerosol-Schaumes vorliegt, so enthält es zusätzlich 2 bis 98 Gewichtsprozent eines Treibmittels und wird in einen Druckbehälter abgefüllt. Als Treibmittel sind beispielsweise leicht flüchtige Fluorchlorkohlenwasserstoffe, wie zum Beispiel Difluorchlormethan oder Trichlormonofluormethan, Tetrafluordichlorethan oder niedere Alkane, wie zum Beispiel n-Butan, i-Butan und Propan, oder auch Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Verbindungen geeignet. Unter den vorstehend genannten Treibmitteln sind niedere Alkane sowie Mischungen von niederen Alkanen bevorzugt.

Die Herstellung des Mittels kann mit heute üblichen Misch- und Rührapparaturen erfolgen. Im ersten Schritt wird das festigende Polymer oder die Polymermischung in Wasser gelöst. Anschließend werden alle weiteren Komponenten mit Ausnahme des Verdickungsmittels zugefügt. Schließlich wird das Verdickungsmittel zugegeben und gegebenenfalls der zur Fällung des festigenden Polymers oder Polymermischung notwendige pH-Wert eingestellt.

Das erfindungsgemäße Mittel wirkt nicht belastend auf das Haar und ist daher besonders für feines Haar geeignet. Insbesondere als haarfestigendes Mittel bewirkt es eine gute Haltbarkeit der Frisur, ohne daß das Haar verklebt oder durch einen hohen Anteil festigender Polymere belastet wird. Feines Haar bekommt Fülle und Volumen. Als haarfestigendes Gel zeigt das Haarbehandlungsmittel bessere festigende Eigenschaften als herkömmliche Gele. Das Mittel ist frei von organischen Lösungsmitteln und wirkt daher weniger belastend auf Mensch und Umwelt.

Folgende Beispiele sollen den Gegenstand der Erfindung näher erläutern:

**Beispiele**

**Beispiel 1: Festigungslotion**

| | |
|---|---|
| 1,00 g | mit Decadien vernetztes Methylvinylether/Maleinsäureanhydrid-Copolymer (Stableze 06®, ISP, England) |
| 3,50 g | Octylacrylamid/Acrylsäure/Butylaminoethylmethacrylat-Copolymer (Amphomer® der Firma National Starch, USA) |
| 2,18 g | Aminomethylpropanol |
| 0,20 g | mit 40 Mol Ethylenoxid oxethyliertes hydriertes Rizinusöl |
| 0,20 g | p-Hydroxybenzoesäuremethylester |
| 0,20 g | Parfümöl |
| 92,72 g | Wasser |
| 100,00 g | |

Die Festigungslotion weist einen pH-Wert von 6,5 auf. Die Viskosität des Mittels beträgt 110 mPa·s, gemessen mit einer Haake-Viskowaage bei 30 Grad Celsius, Stab II und einem Auflagegewicht von 10 g.

**Beispiel 2: Creme-Gel mit normaler Festigung**

| | |
|---|---|
| 6,96 g | Maisstärke |
| 1,70 g | Vinylacetat/Crotonsäure-Copolymer (Luviset CA 66®, BASF, Deutschland) |
| 0,15 g | Kaliumhydroxid |
| 0,30 g | mit 40 Mol Ethylenoxid oxethyliertes hydriertes Rizinusöl |
| 0,20 g | p-Hydroxybenzoesäuremethylester |
| 0,15 g | Parfümöl |
| 90,54 g | Wasser |
| 100,00 g | |

Der pH-Wert des Mittels wird mit Zitronensäure auf 6,5 eingestellt.

**Beispiel 3: Schaumfestiger mit normaler Festigung**

| | |
|---|---|
| 1,2 g | Hydroxyethylcellulose |
| 1,7 g | Vinylacetat/Crotonsäure-Copolymer (Resyn 28-1310®, National Starch, USA) |
| 0,3 g | Aminomethylpropanol |
| 0,2 g | mit 40 Mol Ethylenoxid oxethyliertes hydriertes Rizinusöl |
| 0,2 g | p-Hydroxybenzoesäuremethylester |
| 0,1 g | Parfümöl |
| 96,3 g | Wasser |
| 100,0 g | |

Der pH-Wert des Mittels wird mit Zitronensäure auf 6,5 eingestellt. Anschließend wird das Mittel mit einem Propan/Butan Treibgas-Gemisch (1,5 bar) in einem Gewichtsverhältnis von 95:5 (Mittel:Treibgas) in einen Druckgasbehälter abgefüllt.

**Beispiel 4: Haarkonditionierungsmittel in Schaumform**

| | |
|---|---|
| 1,0 g | Acrylsäurehomopolymer |
| 1,8 g | Acrylsäure/Ethylacrylat/N-tert.-Butylacrylamid-Copolymer (Ultrahold 8®, BASF, Deutschland) |
| 0,8 g | Aminomethylpropanol |
| 0,3 g | mit 35 Mol Ethylenoxid oxethyliertes Rizinusöl |
| 0,4 g | Vinylpyrrolidon/Dimethylaminomethacrylat-Copolymer (Gafquat 755 N®, GAF, USA) |
| 0,3 g | p-Hydroxybenzoesäuremethylester |
| 0,2 g | Parfümöl |
| 95,2 g | Wasser |
| 100,0 g | |

Der pH-Wert des Mittels wird mit Zitronensäure auf 6,5 eingestellt. Anschließend wird das Mittel mit einem Propan/Butan Treibgas-Gemisch (1,5 bar) in einem Gewichtsverhältnis von 95:5 (Mittel:Treibgas) in einen Druckgasbehälter abgefüllt.

**Beispiel 5: Creme-Gel mit starker Festigung**

| | |
|---|---|
| 6,96 g | Maisstärke |
| 3,50 g | Vinylacetat/Crotonsäure-Copolymer (Luviset CA 66®, BASF, Deutschland) |
| 0,30 g | Kaliumhydroxid |
| 0,30 g | mit 40 Mol Ethylenoxid oxethyliertes hydriertes Rizinusöl |
| 0,20 g | p-Hydroxybenzoesäuremethylester |
| 0,20 g | Parfümöl |
| 88,54 g | Wasser |
| 100,00 g | |

Der pH-Wert des Mittels wird mit Zitronensäure auf 6,5 eingestellt.

**Beispiel 6: Gel-Spray**

| | |
|---|---|
| 1,20 g | Hydroxyethylcellulose |
| 1,50 g | Vinylacetat/Crotonsäure-Copolymer (Resyn 28-1310®, National Starch, USA) |
| 0,24 g | Aminomethylpropanol |
| 0,30 g | mit 35 Mol Ethylenoxid oxethyliertes Rizinusöl |
| 0,30 g | p-Hydroxybenzoesäuremethylester |
| 0,20 g | Parfümöl |
| 96,26 g | Wasser |
| 100,00 g | |

Der pH-Wert des Mittels wird mit Zitronensäure auf 6,3 eingestellt. Das Mittel wird als Non-Aerosol Pumpspray abgefüllt.

**Beispiel 7: Liquid-Creme-Gel mit leichter Festigung**

| | |
|---|---|
| 0,20 g | Acrylsäurehomopolymerisat |
| 0,20 g | Vinylacetat/Crotonsäure-Copolymer (Resyn 28-1310®, National Starch, USA) |
| 0,12 g | Aminomethylpropanol |
| 0,20 g | mit 40 Mol Ethylenoxid oxethyliertes hydriertes Rizinusöl |
| 0,20 g | p-Hydroxybenzoesäuremethylester |
| 0,10 g | Parfümöl |
| 98,98 g | Wasser |
| 100,00 g | |

Der pH-Wert des Mittels beträgt 6,6.

**Beispiel 8: Haarleim**

| | |
|---|---|
| 12,5 g | Hydroxypropylcellulose |
| 0,5 g | Acrylsäurehomopolymerisat |
| 8,0 g | Vinylacetat/Crotonsäure-Copolymer (Resyn 28-1310®, National Starch, USA) |
| 1,0 g | Aminomethylpropanol |
| 0,2 g | mit 14 mol Ethylenoxid oxethyliertes Nonylphenol |
| 0,2 g | p-Hydroxybenzoesäuremethylester |
| 0,1 g | Parfümöl |
| 77,5 g | Wasser |
| 100,0 g | |

Der pH-Wert des Mittels beträgt 6,5.

**Beispiel 9: Festigende Kur**

| | |
|---|---|
| 0,5 g | Acrylsäurehomopolymerisat |
| 2,6 g | Vinylacetat/Crotonsäure-Copolymer (Resyn 28-1310®, National Starch, USA) |
| 0,3 g | Aminomethylpropanol |
| 2,2 g | Cetylstearylalkohol (1:1) |
| 0,4 g | Natriumcetylstearylsulfat |
| 0,8 g | mit 2 Mol Ethylenoxid oxethylierter Laurylalkohol |

| | |
|---|---|
| 2,1 g | Vaseline |
| 0,4 g | p-Hydroxybenzoesäuremethylester |
| 0,2 g | Parfümöl |
| 90,5 g | Wasser |
| 100,0 g | |

Der pH-Wert des Mittels beträgt 6,5.

**Beispiel 10: Festigendes Shampoo**

| | |
|---|---|
| 1,2 g | Acrylsäurehomopolymerisat |
| 3,0 g | Vinylacetat/Crotonsäure-Copolymer (Resyn 28-1310®, National Starch, USA) |
| 1,5 g | Aminomethylpropanol |
| 35,0 g | Laurylethersulfat (28 %ige wäßrige Lösung) |
| 10,0 g | Cocoamidopropylbetain |
| 0,2 g | Parfümöl |
| 49,1 g | Wasser |
| 100,0 g | |

Der pH-Wert des Mittels beträgt 6,4.

**Beispiel 11: Tönungsfestiger**

| | |
|---|---|
| 0,20 g | mit Decadien vernetztes Methylvinylether/Maleinsäureanhydrid-Copolymer (Stabilize 06®, ISP, USA) |
| 2,20 g | Vinylacetat/Crotonsäure-Copolymer (Luviset CA 66®, BASF, Deutschland) |
| 0,19 g | Kaliumhydroxid |
| 0,20 g | mit 35 Mol Ethylenoxid oxethyliertes Rizinusöl |
| 0,20 g | p-Hydroxybenzoesäuremethylester |
| 0,05 g | Acid Brown 4 (C.I. 41 805) |
| 0,20 g | Parfümöl |
| 96,76 g | Wasser |
| 100,00 g | |

Der pH-Wert des Mittels beträgt 6,3.

**Patentansprüche**

1. Haarbehandlungsmittel mit einem Gehalt an

   (A) 0,1 bis 30 Gewichtsprozent mindestens eines organischen und/oder anorganischen Verdickers,

   (B) 0,1 bis 25 Gewichtsprozent mindestens eines festigenden wasserlöslichen Polymers, das durch Änderung des pH-Wertes ganz oder teilweise ausfällbar ist, und

   (C) 45 bis 99,8 Gewichtsprozent Wasser, welches

   (D) frei von organischen Lösungsmitteln ist und

   (E) einen pH-Wert aufweist, bei dem die Komponente (B) ganz oder teilweise ausgefällt vorliegt.

2. Mittel nach Anspruch 1, dadurch gekennzeichnet, daß es 0,15 bis 15 Gewichtsprozent der Komponente (A) enthält.

3. Mittel nach Anspruch 1 oder 2, dadurch gekennzeichnet, daß es 0,2 bis 10 Gewichtsprozent der Komponente (B) enthält.

4. Mittel nach einem der Ansprüche 1 bis 3, dadurch gekennzeichnet, daß es 55 bis 96 Gewichtsprozent der Komponente (C) enthält.

5. Mittel nach einem der Ansprüche 1 bis 4, dadurch gekennzeichnet, daß es als Komponente (A) mindestens einen natürlichen, modifizierten natürlichen und/oder synthetischen Verdicker enthält.

6. Mittel nach einem der Ansprüche 1 bis 5, dadurch gekennzeichnet, daß es als Verdicker der Komponente (A) Polymere und Copolymere der Acrylsäure, Polymere und Copolymere der Methacrylsäure, Polymere und Copolymere der Crotonsäure, Polymere und Copolymere der Salze oder Ester der Acrylsäure, Methacrylsäure oder Crotonsäure, mit Dienen vernetzte Copolymere aus ungesättigten Säureanhydriden und Alkylvinylethern, Polyethylenglykole, Polypropylenglykole, Polyethylenglykol-Polypropylenglykol-Copolymere, Polysaccharide, Derivate oder Hydrolysate der Polysaccharide, Gelatine, Hectorite oder Bentonite enthält.

7. Mittel nach einem der Ansprüche 1 bis 6, dadurch gekennzeichnet, daß das festigende wasserlösliche Polymer der Komponente (B) ein natürliches, modifiziertes natürliches oder synthetisches filmbildendes Polymer ist.

8. Mittel nach einem der Ansprüche 1 bis 7, dadurch gekennzeichnet, daß das festigende Polymer der Komponente (B) in Form seines Salzes wasserlöslich, als freie Base und/oder Säure wasserunlöslich ist.

9. Mittel nach einem der Ansprüche 1 bis 8, dadurch gekennzeichnet, daß es als festigendes Polymer der Komponente (B) Copolymere, Pfropfcopolymere oder Terpolymere enthält, die als einen Monomerbaustein Verbindungen aus der Gruppe der ungesättigten Monocarbonsäuren, der ungesättigten Dicarbonsäuren oder der Anhydride von ungesättigten Dicarbonsäuren, und als weiteren Monomerbaustein eine ungesättigte organische Verbindung enthalten.

10. Mittel nach einem der Ansprüche 1 bis 9, dadurch gekennzeichnet, daß es einen pH-Wert von 1,5 bis 11,5 aufweist.

**Claims**

1. Hair treatment agent containing:

    (A) from 0.1 to 30% by weight of at least one organic and/or inorganic thickener;

    (B) from 0.1 to 25% by weight of at least one fixing water-soluble polymer which can be precipitated completely or partially by changing the pH; and

    (C) from 45 to 99.8% by weight of water, which agent

    (D) is free from organic solvents; and

    (E) has a pH at which component (B) is in a completely or partially precipitated form.

2. Agent according to Claim 1, characterised in that it contains from 0.15 to 15% by weight of component (A).

3. Agent according to Claim 1 or 2, characterised in that it contains from 0.2 to 10% by weight of component (B).

4. Agent according to any one of Claims 1 to 3, characterised in that it contains from 55 to 96% by weight of component (C).

5. Agent according to any one of Claims 1 to 4, characterised in that it contains at least one natural, modified natural and/or synthetic thickener as component (A).

6. Agent according to any one of Claims 1 to 5, characterised in that it contains, as thickener of component (A), polymers and copolymers of acrylic acid, polymers and copolymers of methacrylic acid, polymers and copolymers of crotonic acid, polymers and copolymers of the salts or esters of acrylic acid, methacrylic acid or crotonic acid, copolymers that comprise unsaturated acid anhydrides and alkyl vinyl ethers and that have been crosslinked with dienes, polyethylene glycols, polypropylene glycols, polyethylene glycol/polypropylene glycol copolymers, polysaccharides, derivatives or hydrolysates of polysaccharides, gelatins, hectorites or bentonites.

7. Agent according to any one of Claims 1 to 6, characterised in that the fixing water-soluble polymer of component (B) is a natural, modified natural or synthetic film-forming polymer.

8. Agent according to any one of Claims 1 to 7, characterised in that the fixing polymer of component (B) is water-soluble when in the form of its salt and water-insoluble when in the form of the free base and/or acid.

9. Agent according to any one of Claims 1 to 8, characterised in that it contains, as fixing polymer of component (B), copolymers, graft copolymers or terpolymers which contain compounds from the group of the unsaturated monocarboxylic acids, the unsaturated dicarboxylic acids or the anhydrides of unsaturated dicarboxylic acids as one monomer building block and an unsaturated organic compound as further monomer building block.

10. Agent according to any one of Claims 1 to 9, characterised in that it has a pH of from 1.5 to 11.5.

**Revendications**

1. Produit de traitement des cheveux contenant :

    (A) de 0,1 à 30 % en poids d'au moins un épaississant organique et/ou anorganique,

    (B) de 0,1 à 25 % en poids d'au moins un polymère soluble dans l'eau à action renforçante, pouvant être précipité totalement ou partiellement par modification de la valeur du pH, et

    (C) de 45 à 99,8 % en poids d'eau qui

    (D) est exempt de solvants organiques, et

    (E) présente une valeur de pH pour laquelle le composant (B) se présente sous forme totalement ou partiellement précipitée.

2. Produit selon la revendication 1, caractérisé en ce qu'il contient de 0,15 à 15 % en poids du composant (A).

3. Produit selon la revendication 1 ou 2, caractérisé en ce qu'il contient de 0,2 à 10 % en poids du composant (B).

4. Produit selon l'une des revendications 1 à 3, caractérisé en ce qu'il contient de 55 à 96 % en poids du composant (C).

5. Produit selon l'une des revendications 1 à 4, caractérisé en ce qu'il contient comme composant (A) au moins un épaississant naturel, naturel modifié et/ou synthétique.

6. Produit selon l'une des revendications 1 à 5, caractérisé en ce qu'il contient comme épaississant du composant (A), des polymères et de copolymères de l'acide acrylique, des polymères et des copolymères de l'acide méthacrylique et des polymères et des copolymères de l'acide crotonique, des polymères et des copolymères des sels ou des esters de l'acide acrylique, de l'acide méthacrylique ou de l'acide crotonique, des copolymères réticulés à l'aide de diènes et issus d'anhydrides d'acide et d'esters d'alkylvinyle insaturés, des polyéthylèneglycols, des polypropylèneglycols, des copolymères de polyéthylèneglycol-polypropylèneglycols, des polysaccharidess, des dérivés ou des hydrolysats de polysaccharides, de la gélatine, de l'hectorite ou de la bentonite.

7. Produit selon l'une des revendications 1 à 6, caractérisé en ce que le polymère soluble dans l'eau à action renforçante, du composant (B) est un polymère filmogène, naturel, naturel modifié ou synthétique.

8. Produit selon l'une des revendications 1 à 7, caractérisé en ce que le polymère à action renforçante du composant (B) est soluble dans l'eau sous forme de son sel, est insoluble dans l'eau sous forme de base libre et/ou d'acide.

9. Produit selon l'une des revendications 1 à 8, caractérisé en ce qu'il contient comme polymère renforçant du composant (B), des copolymères, des copolymères greffés ou des terpolymères, contenant comme composant monomère des combinaisons du groupe des monoacides carboniques insaturés, des diacides carboniques insaturés ou de l'anhydride de diacides carboniques insaturés et, comme autre composant monomère, un composé organique insaturé.

10. Produit selon l'une des revendications 1 à 9, caractérisé en ce qu'il présente une valeur de pH de 1,5 à 11,5.